# EUROPEAN PATENT SPECIFICATION

(11) **EP 3 807 649 B1**
(45) Date of publication and mention of the grant of the patent: **15.10.2025**
(21) Application number: 19818594.4
(22) Date of filing: 12.06.2019
(51) Int. Cl.: G01N 33/84, G01N 21/82, C01B 9/06, C01G 7/00, G01N 33/50

(54) **METHOD AND KIT FOR QUICK AND LOW-COST DETECTION OF IODIDE IONS IN AQUEOUS SOLUTIONS**
VERFAHREN UND KIT ZUM SCHNELLEN UND KOSTENGÜNSTIGEN NACHWEIS VON IODIDIONEN IN WÄSSRIGEN LÖSUNGEN
PROCÉDÉ ET KIT POUR DÉTECTION RAPIDE ET ÉCONOMIQUE D'IONS IODURES DANS DES SOLUTIONS AQUEUSES

(30) Priority: 15.06.2018 HU 1800212
(43) Date of publication of application: 21.04.2021
(73) Proprietor: IOI Auranae Kft., 1068 Budapest (HU)
(72) Inventor: Köntös, Zoltan, 1112 Budapest (HU)
(74) Representative: Szentpéteri, Zsolt
(86) International application number: PCT/HU2019/000020
(87) International publication number: WO 2019/239168

(56) References cited:
- EP-B1- 1 133 346
- CN-A- 103 018 238
- RU-C2- 2 279 681
- RU-C2- 2 377 557
- PHILLIP SHELOR C ET AL: "Review of analytical methods for the quantification of iodine in complex matrices", ANALYTICA CHIMICA ACTA, ELSEVIER, AMSTERDAM, NL, vol. 702, no. 1, 25 May 2011 (2011-05-25), pages 16 - 36, XP028252995, ISSN: 0003-2670, [retrieved on 20110623], DOI: 10.1016/J.ACA.2011.05.039
- THEODORE T ZAVA ET AL: "Assessment of Japanese iodine intake based on seaweed consumption in Japan: A literature-based analysis", THYROID RESEARCH, BIOMED CENTRAL LTD, LONDON, UK, vol. 4, no. 1, 5 October 2011 (2011-10-05), pages 14, XP021111282, ISSN: 1756-6614, DOI: 10.1186/1756-6614-4-14
- HAND DARROW ET AL: "Iodine or Iodide? A Laboratory Evaluation of the Content of Powdered Iodine Supplements", vol. 6, no. 1, 1 December 2017 (2017-12-01), pages 62 - 68, XP055873667, ISSN: 2165-7971, Retrieved from the Internet <URL:https://restorativemedicine.org/wp-content/uploads/2017/12/Evaluation-of-the-Content-of-Powdered-Iodine-Supplements.pdf> DOI: 10.14200/jrm.2017.6.0106
- KIENER C ET AL: "High-throughput screening under demanding conditions: Cu/ZnO catalysts in high pressure methanol synthesis as an example", JOURNAL OF CATALYSIS, ACADEMIC PRESS, DULUTH, MN, US, vol. 216, no. 1, 6 March 2003 (2003-03-06), pages 110 - 119, XP085067303, ISSN: 0021-9517, DOI: 10.1016/S0021-9517(02)00134-3

## Description

### BACKGROUND OF THE INVENTION

### 1. Technical Field of the invention

Iodine is an essential building block of two thyroid hormones (thyroxine and triiodothyronine). Lack of iodine causes the enlargement of the thyroid tissue (endemic goiter), a decrease in physical/mental performance during puberty, irreversible brain damage in the fetal stage, decrease in learning abilities and IQ. At the same time, excessive intake of iodine may result in thyroid dysfunction.

The importance and topicality of the topic are demonstrated by the fact that in the autumn of 1990, 151 Heads of State (or its representatives) signed the UN's agreement which declare in its action plan that iodine deficiency should be eliminated worldwide. The international picture is strongly negative because 97 million people with struma and about 900,000 people with mentally disabilities (cretenism) suffer from the consequences of iodine deficient nutrition in Europe (WHO / UNICEF / ICCIDD 1993). Iodine deficiency is one of the most common causes of the preventable mental retardation. Iodine deficiency affects the reproductive function (Glinoer, D. (1997): Clinical Obstetrics and Ginecology, 40: pp. 102-116) and children's learning abilities, as well as the severe iodine deficiency can reduce IQ levels by up to 13.5 points. (Dunn, JT, Delange, F. (2001) Journal of Clinical Endocrinology and Metabolism, 86: pp. 2360-2363).

Iodine enters into our body with food and drinking water. The iodide ion is rapidly absorbed and then accumulated in the thyroid gland. Excess amounts are secreted into the urine, but also appeared in saliva and breast milk.

The iodide ion level is determined by 24-hour urine collection as the body excretes the excess of the iodine.

The invention relates to a method and use of a kit for quick and low-cost determination of the iodide ion content, which can be used not only to detect the possible iodine deficiency conditions but also to estimate the iodide ion concentration of liquids. The scope of protection is defined by the appended claims.

### 2. Description of the Prior Art

Several methods are known for determining iodide ion. A number of solutions existed for determining iodide ions from classical titration procedures to more complex ion chromatography methods. However, these procedures assume well-equipped laboratory conditions and include time-consuming procedures. The cost of such detection procedures is significant. Methods are known in which iodide ions are measured from urine by ion pair chromatography with electrochemical detection systems (Below H. et al., Fresenius J. Anal Chem. 2001 Oct; 371 (4): 431-6). The most frequently used clinical method for the detection of iodide ion is inductively coupled plasma mass spectrometry. Although the corresponding process provides rapid and accurate quantified data, the sample preparation and the measurement require a great expertise and substantial financial effort. The process can only be performed under laboratory conditions.

RU 2325658 discloses an ionometric method of analysis wherein the determination of the iodide ion concentration is based on an iodide ion selective electrode potential. Implementing the procedure by doing at home is extremely complicated and time consuming. Purchasing the necessary tools involves significant costs.

CN 103018238 discloses a rapid and effective method for determining the concentration of iodide ions. Metal (gold and silver) nanocomposite materials with good water solubility and high molar absorptivity are produced and the oxidative aggregation of which are inhibited by iodide ions. Thus, the iodide ion content in the admixed solution can be determined by colorimetric assay.
Kiener C. et al "High-Throughput screening under demanding conditions: Cu/ZnO catalysts in high pressure methanol synthesis as an example" (Journal of Catalysts, Academic Press, Duluth, MN, US, vol. 216, no. 1, 6 March 2003 (2003-03-06), pages 110-119, XP085067303, ISSN: 0021-9517, DOI: 10.1016/S0021-9517(02)00134-3 discloses, in combination a 0,7 mol/L Cu(NO₃)₂ solution and a polypropylene vessel having a volume of at least 20 ml.

### SUMMARY OF THE INVENTION

We have found that the above-mentioned methods allow accurate iodide concentration determination, however, their need for instruments and expertise are significant, so they are not suitable for doing at home iodide determination. In addition, the cost of the above procedures is significant, so their wide availability is limited. The above procedures are not applicable as a quick test, and are therefore inaccessible in poor countries of the world. Given that iodine deficiency affects a significant portion of the Earth's population, there is a strong need to develop a rapid test that will allow to determine the daily iodine needs of the body of their own and their families for a significant number of people in a simple procedure to avoid the aforementioned diseases.

In our research, it has been discovered that the iodide ion and an aqueous solution of water-soluble metal salts with a suitably chosen concentration in normal condition for detecting iodide ion [I⁻] in aqueous solutions, solution samples, especially in human and animal urine selectively form a precipitate. By adding a solution, which comprising metal ion, preferably a gold ion or a copper ion and selectively precipitates iodide ion to the sample, the amount of the iodide ion in the aqueous solution or such samples, especially in human and animal urine can be measured based on the colour and consistency of the water-insoluble iodide salt precipitate formed during the precipitation in the aqueous solution or such samples, especially in human and animal urine, as well as, the metal concentration needed for the precipitation and the time needed for the starting of the precipitation.

The present invention is based on the forming of copper(I) iodide in two steps by adding an aqueous solution of copper salts to the iodide ions exist in the urine in dissolved form, which is a water-insoluble precipitate (0.00042 g / L at 25 ° C) and easily detectable by eye. Unexpectedly, it has been found that the copper salts selectively form precipitates with iodide ions dissolved in the urine, which provides the option of selective detection of iodide ions. The metal salt concentrations required for the detection of iodide ions can be calculated from the solubility product constant typical for the resulting precipitates.

The metal-ion containing solutions produced from water-soluble metal salts and by selecting a suitable metal concentration selectively precipitate iodide ion under normal condition are readily available, directly non-toxic to human body and thus these can be used extensively and safely for the rapid test to be claimed. Such metal ions may include, in particular, copper (II) ions and gold (III) ions.

In the aqueous solution used in the process of the invention, the metal ion content is provided in the form of water-soluble metal salts. Examples of such metal salts include CuSO₄, Cu(NO₃)₂, Cu(CH₃COO)₂, and crystalline hydrated forms thereof, e.g. CuSO₄.5H₂O, Cu(NO₃)₂.2.5H₂O, Cu(NO₃)₂.3H₂O or Cu(NO₃).2.5H₂O_{.} According to the invention Cu(NO3)2 is used.

Determination of iodide ion concentration by gold ion is a classical titration process, but it can be stated in the case of gold ions that selective precipitation occurs in the urine under suitably selected conditions, thus iodide ion concentration can be easily determined by this process. An aqueous metal salt solution of the gold (III) ions is prepared from AuCl₃ and crystalline hydrated forms from AuCl₃.H2O and AuCl₃.3H₂O.

### DETAILED DESCRIPTION OF THE INVENTION

The present invention relates to a method for the determination of iodide ions as claimed. In general the method can be described as a method for the determination of iodide ions in an aqueous solution, comprising the steps of:
a) providing a liquid sample for which iodide ion concentration has to be determined,
b) adding at least one drop of aqueous solution of a metal ion to the liquid sample of step a) wherein the metal ions selectively precipitate with iodide ion under normal conditions;
c) shaking the liquid sample of step b) and waiting for 1 to 5 minutes, preferably 3 minutes;
d) repeating steps b) and c) until the precipitation is clearly visible to the naked eye in the test solution;
e) recording and summing up the number of drops; and
f) determining the amount of iodide ions based on the number of drops.

The present invention relates to a method for the determination of iodide ions in an aqueous solution, wherein said method further comprises step a1) after step a), wherein the liquid sample is allowed to settle for 10 to 15 minutes and the supernatant liquid is separated from the settled portion.

The present invention also relates to a method for the determination of iodide ions in an aqueous solution, wherein said method further comprises step a2) after step a), wherein the liquid sample is filtered to filter out the floating impurities or protein aggregates prior to the assay.

The present disclosure also relates to a method for the determination of iodide ions in an aqueous solution, wherein the concentration of the metal-salt solution is ranging from 10⁻⁷ to 10⁴ mol/l, preferably from 10⁻² to 10² mol/l, more preferably from 0.1 to 1 mol/l.

The present invention further relates to the use of a kit for determination of iodide ions in an aqueous solution. The kit comprises a sample vessel, a vessel equipped with a dropper wherein the said vessel contains a metal salt solution which selectively precipitates with at least one iodide ion under normal conditions and an instruction for describing the method of determining the iodide ions in aqueous solution.

The aqueous solution to be tested may be any aqueous solution such as drinking water, milk, urine, preferably human or animal urine.

The method according to the invention should be used with caution, because if a person has high levels of iodide in the urine, it can even give a brownish, cloudy precipitate after the first drop. In relation to drop numbers the precipitation is expected within the range of 1 to 15 drops. It is necessary to observe the waiting time between the droplets, because the brownish precipitation develops over time and not immediately. The waiting time can be 1 to 5 minutes, preferably 3 minutes.

After carrying out the method according to the invention, the said sample can be treated with potassium ferrocyanide (K₄Fe(CN)₆), thus contents of the sample can be disposed of in the sewer system.

The urine sample to be tested in the method according to the invention may be a 24-hour collection or the first or second urine sample of the morning. If the aqueous solution to be tested is urine and the objective is to determine the iodine deficient condition, iodine deficient condition is determined by the following correlation: 1 to 2 drops of metal ion solution mean the optimal iodide concentration (100 µg/l), 3 to 5 drops of metal ion solution mean mild iodine deficiency (50 to 99 µg/l), 6 to 7 drops of metal ion solution mean moderate iodine deficiency (20 to 49 µg/l), while 8 or more drops mean severe iodine deficiency (<20 µg/l).

The quick test for iodine secreted with urine determines values for different ranges. The daily iodine level fluctuation is related to the nutrition, therefore to be able to get the most accurate picture of the iodine supply of the subject who provided the sample, it is recommended to repeat the test once a week at the same time for a month and to evaluate the average of the resulting number of drops.

The kit used in the method according to the present invention also comprises an electronic application available on an electronic network, preferably via the Internet, which can be used to continuously monitor the subject's iodide ion level based on the values entered. This allows the individual to control the amount of iodine being delivered to the body.

### EXAMPLES

### Example 1. Determination of iodide ion content from urine by solution of Cu(NO₃)₂.

At least 20 ml of urine sample was collected from the first or second urine in the morning, which was filled into a container with a transparent, quantitative mark and screw cap up to the indicated amount (20 ml). In the plastic reservoir sample holder, the urine, which can be observed in the various shades of yellow is a clear, translucent liquid by default.

The collected sample was cloudy, not completely transparent, so it was allowed to settle at low temperatures. After settling, the top of the urine was filled into the sample holder up to the mark. Then, a solution containing 0.5 mol/l Cu(NO₃)₂ provided in the kit separately in a dropper vial was added dropwise until the otherwise pure urine began to cloud and a brownish-brown precipitate was observed. After each drop, the urine sample was shaken and waited 1 minute before adding the following drop. After the first drop was added, a colour change occurred and the yellow urine began to green. The change in colour does not affect the measurement, only the formation of the brownish precipitate was observed. After the urine began to get opalescent, but the precipitate had not yet precipitated, one drop was needed until the observation of the precipitation by naked eye, which meant the end of the measurement.

The concentration of iodide ion in the urine is determined by the number of drops added before the development of the brownish precipitate. The more drops are needed, the less iodine is secreted by the body.

The analysis of the precipitate showed that the sample solution contained only CuI precipitate as a precipitate. Thus, the copper nitrate solution is suitable for the selective detection of iodide ion from urine.

Table 1. shows the correlation between the number of drops and the iodide ion concentration.

**Table 1. Ranges of iodide ion concentration**

| Concentration of iodide ion | Stages of iodine deficiency | The amount of the test solution which needed to precipitation of the cloudy, brownish precipitate |
|---|---|---|
| more than 99 microgram/litre | optimal | 1 to 2 drops |
| 50 to 99 microgram/litre | mild iodine deficiency | 3 to 5 drops |
| 49,8 to 20 microgram/litre | moderate iodine deficiency | 6 to 7 drops |
| less than 20 microgram/litre | severe iodine deficiency | more than 8 drops |

### Example 2. Determination of iodide ion content from urine by solution of CuSO₄.

At least 20 ml of urine sample was collected from the first or second urine in the morning, which was filled into a container with a transparent, quantitative mark and screw cap up to the indicated amount (20 ml). In the plastic reservoir sample holder, the urine, which can be observed in the various shades of yellow is a clear, translucent liquid by default.

The collected sample was cloudy, not completely transparent, so it was allowed to settle at low temperatures. After settling, the top of the urine was filled into the sample holder up to the mark. Then, a solution containing 0.7 mol/l CuSO₄ provided in the kit separately in a dropper vial was added dropwise until the otherwise pure urine began to cloud and a brownish-brown precipitate was observed. After each drop, the urine sample were shaken and waited 1 minute before adding the following drop. After the first drop was added, a colour change occurred and the yellow urine began to green. The change in colour does not affect the measurement, only the formation of the brownish precipitate was observed. After the urine began to get opalescent, but the precipitate had not yet precipitated, one drop was needed until the observation of the precipitation by naked eye, which meant the end of the measurement.

The concentration of iodide ion in the urine is determined by the number of drops added before the development of the brownish precipitate. The more drops are needed, the less iodine is secreted by the body.

The analysis of the precipitate showed that the sample solution contained only CuI precipitate as a precipitate. Thus, the copper sulphate solution is suitable for the selective detection of iodide ion from urine.

### Example 3. Determination of iodide ion content from urine by solution of AuCl₃.

At least 20 ml of urine sample was collected from the first or second urine in the morning, which was filled into a container with a transparent, quantitative mark and screw cap up to the indicated amount (20 ml). In the plastic reservoir sample holder, the urine, which can be observed in the various shades of yellow is a clear, translucent liquid by default.

The collected sample was cloudy, not completely transparent, so it was allowed to settle at low temperatures. After settling, the top of the urine was filled into the sample holder up to the mark. Then, a solution containing 0.2 mol/l AuCl₃ provided in the kit separately in a dropper vial was added dropwise until the otherwise pure urine began to cloud and a brownish-brown precipitate was observed. After each drop, the urine sample was shaken and waited 1 minute before adding the following drop. After the first drop was added, a colour change occurred and the yellow urine began to green. The change in colour does not affect the measurement, only the formation of the brownish precipitate was observed. After the urine began to get opalescent, but the precipitate had not yet precipitated, one drop was needed until the observation of the precipitation by naked eye, which meant the end of the measurement.

The concentration of iodide ion in the urine is determined by the number of drops added before the development of the brownish precipitate. The more drops are needed, the less iodine is secreted by the body.

The analysis of the precipitate showed that the sample solution contained only CuI precipitate as a precipitate. Thus, the gold chloride solution is suitable for the selective detection of iodide ion from urine.

### Comparative Example 1.

During the calibration of the number of droplets, the iodide ion concentration was determined in mg/L units for 5 parallel measurements of the same urine by ICP-MS technique, which was used to calculate the theoretical concentration of the copper solution based on the solubility product constant to determine the required concentration. In addition to the copper concentration used, the reagent consumption was inversely proportional to the concentration of iodide ions. The fact that the precipitate was copper (I) iodide was detected by ICP-MS.

## Claims

1. A method for quick and low-cost determination of iodide ions in urine, comprising the steps of:
a) providing an urine sample of 20 ml for which iodide ion concentration has to be determined,
b) adding at least one drop of aqueous solution of Cu(NO₃)₂ or their hydrated forms to the liquid sample of step a) wherein Cu-ion selectively precipitates with iodide ion under normal conditions and has a concentration of 0.2 to 0.7 mol/l;
c) shaking the liquid sample of step b) and waiting for 1 to 10 minutes;
d) repeating steps b) and c) until the precipitation is clearly visible to the naked eye in the test solution;
e) recording and summing up the number of droplets; and
f) determining the amount of iodide ions based on the number of drops,
wherein 1 to 2 drops of metal ion solution mean the optimal iodide concentration (>99 µg/l), 3 to 5 drops of metal ion solution mean mild iodine deficiency (50 to 99 µg/l), 6 to 7 drops of metal ion solution mean moderate iodine deficiency (20 to 49 µg/l), while 8 or more drops mean severe iodine deficiency (<20 µg/l).

2. The method according to claim 1, **characterized in that** said method comprises a further step of a1) after step a), wherein the liquid sample is allowed to settle for 10 to 15 minutes and the supernatant liquid is separated from the settled portion.

3. The method according to claim 2, **characterized in that** said method comprises a further step of a2) after step a1), wherein the liquid sample is cooled and filtered to filter out the floating impurities or protein aggregates prior to the assay.

4. The method according to anyone of claims 1 to 3, **characterized in that** the said liquid sample is a human or animal urine.

5. The method according to anyone of claims 1 to 4, **characterized in that** 2 to 5 minutes wait is required between the drops.

6. The method according to claim 1 to 5, **characterized in that** the method comprises a further step for treating the sample with potassium ferrocyanide (K₄Fe(CN)₆) after finishing the determination, thus contents of the sample can be disposed of in the sewer system.

7. The use of a kit for accomplishing the method according to any of claims 1 to 6, wherein the kit comprises a sample vessel of 20 ml, a vessel equipped with a dropper wherein the said vessel contains a 0.2 to 0.7 mol/l Cu(NO₃)₂ or their hydrated form solution and an instruction for describing the method of determining the iodide ions in urine sample.

8. The use according to claim 7, wherein the kit further comprises a solution of potassium ferrocyanide (K₄Fe(CN)₆) for treating the determined sample to be able to dispose of it into the sewer system.

9. The use according to claims 7 or 8 for quick and low-cost determination of iodide ion concentration in urine samples.

10. The use according to claims 7 or 8 for continuous monitoring of the iodide deficient condition in a subject.

11. The use according to claim 10, wherein the monitoring is carried out manually.

12. The use according to claim 10, wherein the monitoring is carried out electronically by computer program.

13. The use according to claim 10, wherein the monitoring is carried out by mobile application.

## Patentansprüche

1. Verfahren zur schnellen und kostengünstigen Bestimmung von Iodidionen in Urin, umfassend die folgenden Schritte:
a) Bereitstellen einer Urinprobe von 20 ml, für die die Iodidionenkonzentration zu bestimmen ist,
b) Zugeben mindestens eines Tropfens einer wässrigen Lösung von Cu(NO₃)₂ oder ihren hydratisierten Formen zu der flüssigen Probe aus Schritt a), wobei Cu-Ion unter normalen Bedingungen selektiv mit Iodidionen ausfällt und eine Konzentration von 0,2 bis 0,7 mol/l aufweist;
c) Schütteln der flüssigen Probe aus Schritt b) und 1 bis 10 Minuten Warten;
d) Wiederholen der Schritte b) und c), bis die Ausfällung in der Testlösung mit bloßem Auge deutlich sichtbar ist;
e) Aufzeichnen und Addieren der Anzahl an Tröpfchen; und
f) Bestimmen der Menge an Iodidionen anhand der Tropfenzahl,
wobei 1 bis 2 Tropfen Metallionenlösung die optimale Iodidkonzentration (> 99 µg/l), 3 bis 5 Tropfen Metallionenlösung einen leichten Iodmangel (50 bis 99 µg/l), 6 bis 7 Tropfen Metallionenlösung einen mittleren Iodmangel (20 bis 49 µg/l) bedeuten, während 8 oder mehr Tropfen einen schweren Iodmangel (<20 µg/l) bedeuten.

2. Verfahren nach Anspruch 1, **dadurch gekennzeichnet, dass** das Verfahren einen weiteren Schritt a1) nach Schritt a) umfasst, wobei man die flüssige Probe 10 bis 15 Minuten absetzen lässt und die überstehende Flüssigkeit von dem abgesetzten Teil getrennt wird.

3. Verfahren nach Anspruch 2, **dadurch gekennzeichnet, dass** das Verfahren einen weiteren Schritt a2) nach Schritt a1) umfasst, wobei die flüssige Probe gekühlt und filtriert wird, so dass die schwebenden Verunreinigungen oder Proteinaggregate vor dem Assay herausgefiltert werden.

4. Verfahren nach einem der Ansprüche 1 bis 3, **dadurch gekennzeichnet, dass** es sich bei der flüssigen Probe um Urin eines Menschen oder Tiers handelt.

5. Verfahren nach einem der Ansprüche 1 bis 4, **dadurch gekennzeichnet, dass** zwischen den Tropfen eine Wartezeit von 2 bis 5 Minuten erforderlich ist.

6. Verfahren nach Anspruch 1 bis 5, **dadurch gekennzeichnet, dass** das Verfahren einen weiteren Schritt zur Behandlung der Probe mit Kaliumhexacyanoferrat(II) (K₄Fe(CN)₆) nach Beendigen der Bestimmung umfasst, sodass sich der Inhalt der Probe in die Kanalisation entsorgen lässt.

7. Verwendung eines Kits zur Bewerkstelligung des Verfahrens nach einem der Ansprüche 1 bis 6, wobei das Kit ein 20-ml-Probengefäß, ein mit einem Tropfer ausgestattetes Gefäß, wobei das Gefäß eine Lösung von 0,2 bis 0,7 mol/l Cu(NO₃)₂ oder ihrer hydratisierten Form enthält, und eine Anweisung zur Beschreibung des Verfahrens zur Bestimmung der Iodidionen in einer Urinprobe umfasst.

8. Verwendung nach Anspruch 7, wobei das Kit ferner eine Lösung von Kaliumhexacyanoferrat (II) (K₄Fe(CN)₆) zur Behandlung der bestimmten Probe umfasst, damit diese in die Kanalisation entsorgt werden kann.

9. Verwendung nach Anspruch 7 oder 8 zur schnellen und kostengünstigen Bestimmung der Iodidionenkonzentration in Urinproben.

10. Verwendung nach Anspruch 7 oder 8 zur kontinuierlichen Überwachung des Iodidmangelzustands bei einem Individuum.

11. Verwendung nach Anspruch 10, wobei die Überwachung manuell erfolgt.

12. Verwendung nach Anspruch 10, wobei die Überwachung elektronisch per Computerprogramm erfolgt.

13. Verwendung nach Anspruch 10, wobei die Überwachung per Mobilanwendung erfolgt.

## Revendications

1. Procédé de détermination rapide et peu coûteuse d'ions iodure dans l'urine, comprenant les étapes de :
a) fourniture d'un échantillon d'urine de 20 ml pour lequel la concentration en ions iodure doit être déterminée,
b ajout d'au moins une goutte de solution aqueuse de Cu(NO₃)₂ ou de leurs formes hydratées à l'échantillon liquide de l'étape a), l'ion Cu précipitant sélectivement avec l'ion iodure dans des conditions normales et ayant une concentration de 0,2 à 0,7 mole/l ;
c) agitation de l'échantillon liquide de l'étape b) et attente 1 à 10 minutes ;
d) répétition des étapes b) et c) jusqu'à ce que la précipitation soit clairement visible à l'œil nu dans la solution d'essai ;
e) enregistrement et somme du nombre de gouttelettes ; et
f) détermination de la quantité d'ions iodure basée sur le nombre de gouttes,
1 à 2 gouttes de solution d'ions métalliques signifiant que la concentration optimale en iodure (> 99 µg/l), 3 à 5 gouttes de solution d'ions métalliques signifiant un déficit léger en iode (50 à 99 µg/l), 6 à 7 gouttes de solution d'ions métalliques signifiant un déficit modéré en iode (20 à 49 µg/l), tandis que 8 gouttes ou plus signifient un déficit sévère en iode (< 20 µg/l).

2. Procédé selon la revendication 1, **caractérisé en ce que** ledit procédé comprend une étape supplémentaire de a1) après l'étape a), dans lequel on laisse l'échantillon liquide décanter pendant 10 à 15 minutes et on sépare le liquide surnageant de la partie décantée.

3. Procédé selon la revendication 2, **caractérisé en ce que** ledit procédé comprend une étape supplémentaire de a2) après l'étape a1), dans lequel l'échantillon liquide est refroidi et filtré pour filtrer les impuretés flottantes ou les agrégats de protéines avant le dosage.

4. Procédé selon l'une quelconque des revendications 1 à 3, **caractérisé en ce que** ledit échantillon liquide est une urine humaine ou animale.

5. Procédé selon l'une quelconque des revendications 1 à 4, **caractérisé en ce que** 2 à 5 minutes d'attente sont nécessaires entre les gouttes.

6. Procédé selon les revendications 1 à 5, **caractérisé en ce que** le procédé comprend une étape supplémentaire pour traiter l'échantillon avec du ferrocyanure de potassium (K₄Fe(CN)₆) après avoir terminé la détermination, de sorte que les contenus de l'échantillon peuvent être éliminés dans le système d'égouts.

7. Utilisation d'un kit pour réaliser le procédé selon l'une quelconque des revendications 1 à 6, dans laquelle le kit comprend un récipient d'échantillon de 20 ml, un récipient équipé d'un compte-gouttes dans lequel ledit récipient contient une solution de Cu(NO₃)₂ à 0,2 à 0,7 mole/l ou leur forme hydratée et une instruction pour décrire le procédé de détermination des ions iodure dans un échantillon d'urine.

8. Utilisation selon la revendication 7, dans laquelle le kit comprend en outre une solution de ferrocyanure de potassium (K₄Fe(CN)₆) pour traiter l'échantillon déterminé afin de pouvoir l'éliminer dans le système d'égouts.

9. Utilisation selon la revendication 7 ou 8 pour la détermination rapide et peu coûteuse de la concentration en ions iodure dans des échantillons d'urine.

10. Utilisation selon la revendication 7 ou 8 pour la surveillance continue de l'état de déficit en iodure chez un sujet.

11. Utilisation selon la revendication 10, dans laquelle la surveillance est effectuée manuellement.

12. Utilisation selon la revendication 10, dans laquelle la surveillance est effectuée électroniquement par un programme informatique.

13. Utilisation selon la revendication 10, dans laquelle la surveillance est effectuée par une application mobile.
